# EUROPEAN PATENT APPLICATION

(11) **EP 3 936 123 A1**
(43) Date of publication of application: **12.01.2022**
(21) Application number: 20766925.0
(22) Date of filing: 05.03.2020
(51) Int. Cl.: A61K 31/01, A61P 1/00, C07F 9/10

(54) **FATTY ACID COMPOSITION AND APPLICATION THEREOF**

(30) Priority: 05.03.2019 CN 201910164602
(71) Applicant: Sichuan Gowell Pharmaceutical Co., Ltd., Meishan City, Sichuan 620010 (CN)
(72) Inventor: SU, Cheng, Meishan City, Sichuan 620010 (CN); DENG, Chongyang, Meishan City, Sichuan 620010 (CN); LUO, Xudong, Meishan City, Sichuan 620010 (CN); LIU, Jing, Meishan City, Sichuan 620010 (CN); XIE, Yu, Meishan City, Sichuan 620010 (CN); GUO, Lixin, Meishan City, Sichuan 620010 (CN); GUO, Hui, Meishan City, Sichuan 620010 (CN)
(74) Representative: Geling, Andrea
(86) International application number: PCT/CN2020/077877
(87) International publication number: WO 2020/177728

(57) **Abstract**

The present application provides a phosphatidylcholine product represented by formula (I) and a fatty acid composition containing same. In formula (I), R₁ and R₂ are each independently selected from residues of saturated or unsaturated fatty acids having more than 12 carbon atoms; and R₁ and R₂ comprise an EPA residue and a DHA residue. The phosphatidylcholine product represented by formula (I) and fatty acid composition provided by the present application can improve damage of IBD model animal colon tissues to a certain extent, have certain anti-IBD activity, and are superior to the first-line drug, sulfasalazine.

## Description

### FIELD OF THE INVENTION

The application belongs to the field of pharmaceutical technology, in particular to a fatty acid composition and use thereof.

### BACKGROUND OF THE INVENTION

Inflammatory bowel disease (IBD) is a type of chronic idiopathic gastrointestinal disease, including ulcerative colitis (UC) and Crohn's disease (CD). At present, there is no specific medicine for the clinical treatment of IBD. The first-line clinical medicine is mesalazine, the highest clinical effective rate of which is only about 50%, and the adverse reactions thereof are serious. Mesalazine is suitable for patients with mild to moderate UC, and patients with severe UC are often treated in combination with hormone medicines and biological medicines. TNF-α biological antibodies and JAK inhibitors which are mainly applied for patients with severe or refractory UC and CD, can also be used for the treatment of IBD. The TNF-α biological antibodies and JAK inhibitors in China, which are expensive, are mainly imported medicines, and all domestic congeneric species are in clinical stages.

n-3 (ω-3 or Ω-3), a type of polyunsaturated fatty acid (PUFA), which is commonly found in deep-sea fishes, is very beneficial to human health. Current studies have shown that pharmaceutical compositions rich in n-3 polyunsaturated fatty acids can be used to treat a variety of clinical indications, such as hypertriglyceridemia and coronary heart disease.

WO2011050474 discloses a therapeutic phospholipid composition for the treatment of cardiometabolic disorders such as hypertriglyceridemia, comprising a compound of Formula (a): 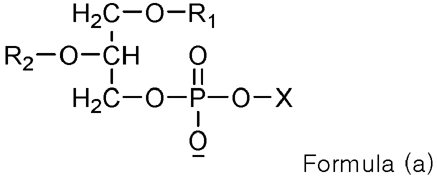 wherein, R₁ and R₂ may be selected from hydrogen or any fatty acid, and X may be an ethanolamine group, a choline group or an inositol group. However, the patent application does not disclose that the phospholipid composition therein can be used for the treatment of inflammatory bowel disease.

WO2014154683 discloses a pharmaceutical preparation, comprising a phosphatidylcholine product of Formula (b): 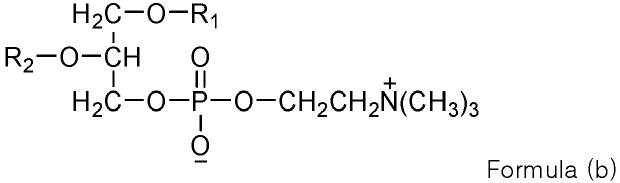 wherein, R₁ and R₂ mainly are linoleic acid, palmitic acid, oleic acid, linolenic acid and stearic acid. The preparation, LT-02, is currently a phase III clinical product, which is used to treat patients with relapsed mild to moderate UC after mesalazine treatment, but the patent application does not mention EPA or DHA.

At present, there is an urgent need to develop medicaments with better efficacy for the treatment of inflammatory bowel disease.

### SUMMARY OF THE INVENTION

In view of this, the object of the present application is to provide a phosphatidylcholine product or a fatty acid composition comprising the same. The fatty acid composition provided in the present application has a better therapeutic effect on the inflammatory bowel disease.

Provided herein is a phosphatidylcholine product represented by Formula (I):

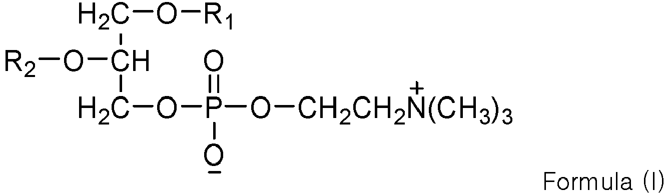

in Formula (I), R₁ and R₂ are each independently selected from residues of saturated or unsaturated fatty acids having 12 or more carbon atoms; and EPA residue(s) and DHA residue(s) are comprised in R₁ and R₂, an area ratio between EPA and DHA is (about 0.33 to about 0.6):1.

Provided herein is a fatty acid composition, comprising the phosphatidylcholine product represented by Formula (I):

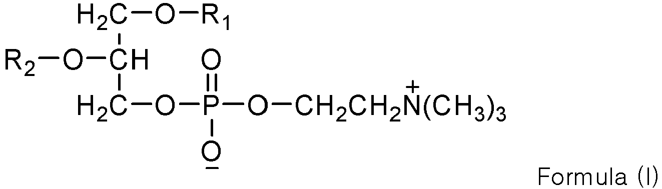

in Formula (I), R₁ and R₂ are each independently selected from residues of saturated or unsaturated fatty acids having 12 or more carbon atoms; and EPA residue(s) and DHA residue(s) are comprised in R₁ and R₂, an area ratio between EPA and DHA is (about 0.33 to about 0.6):1.

Also provided herein is a fatty acid composition, comprising the phosphatidylcholine product represented by Formula (I):

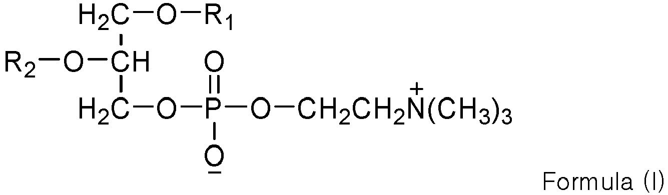

in Formula (I), R₁ and R₂ are each independently selected from residues of saturated or unsaturated fatty acids having 12 or more carbon atoms; and EPA residue(s) and DHA residue(s) are comprised in R₁ and R₂; an area percentage of EPA to a total amount of fatty acids in Formula (I) is about 20% to about 30%; an area percentage of DHA to the total amount of fatty acids in Formula (I) is about 50% to about 60%.

In the phosphatidylcholine product represented by Formula (I), R₁ and R₂ are each independently selected from residues of saturated or unsaturated fatty acids having 12 or more carbon atoms, and EPA residue(s) and DHA residue(s) are comprised in R₁ and R₂.

In certain embodiments, in the phosphatidylcholine product represented by Formula (I), R₁ and R₂ are each independently selected from residues of saturated or unsaturated fatty acids having 12 to 24 carbon atoms. In some preferred embodiments, in the phosphatidylcholine product represented by Formula (I), R₁ and R₂ are each independently selected from residues of saturated or unsaturated fatty acids having 14 to 22 carbon atoms.

In various embodiments, an area ratio of EPA to DHA in the phosphatidylcholine product represented by Formula (I) or the fatty acid composition is (about 0.2 to about 1):1, for example, (about 0.25 to about 1):1, (about 0.26 to about 1):1, (about 0.27 to about 1):1, (about 0.28 to about 1):1, (about 0.29 to about 1):1, (about 0.3 to about 1):1, (about 0.31 to about 1):1, (about 0.32 to about 1):1, (about 0.33 to about 1):1; or for example, (about 0.25 to about 0.95):1, (about 0.25 to about 0.9):1, (about 0.25 to about 0.85):1, (about 0.25 to about 0.8):1, (about 0.25 to about 0.78):1, (about 0.25 to about 0.76):1, (about 0.25 to about 0.75):1, (about 0.25 to about 0.73):1, (about 0.25 to about 0.7):1, (about 0.25 to about 0.69):1, (about 0.25 to about 0.68):1, (about 0.25 to about 0.67):1, (about 0.25 to about 0.66):1, (about 0.25 to about 0.65):1, (about 0.25 to about 0.64):1, (about 0.25 to about 0.63):1, (about 0.25 to about 0.62):1, (about 0.25 to about 0.61):1, (about 0.25 to about 0. 6):1 ; or for example, (about 0.3 to about 0.9):1 , (about 0.3 to 0.85):1, (about 0.3 to about 0.8):1, (about 0.3 to about 0.75):1, (about 0.3 to about 0.7):1, (about 0.3 to about 0.65):1, (about 0.3 to about 0.6):1, (about 0.3 to about 0.59):1 , (about 0.3 to about 0.58):1 , (about 0.3 to about 0.57):1, (about 0.3 to about 0.56):1, (about 0.3 to about 0.55):1. In various embodiments, the area ratio of EPA to DHA in the phosphatidylcholine product represented by Formula (I) or the fatty acid composition is (about 0.33 to about 0.6):1. In certain embodiments, the area ratio of EPA to DHA in the phosphatidylcholine product represented by Formula (I) or the fatty acid composition is (about 0.34 to about 0.38):1; in certain embodiments, the area ratio of EPA to DHA in the phosphatidylcholine product represented by Formula (I) or the fatty acid composition is (about 0.36 to about 0.40):1; in certain embodiments, the area ratio of EPA to DHA in the phosphatidylcholine product represented by Formula (I) or the fatty acid composition is (about 0.38 to about 0.42):1 ; in certain embodiments, the area ratio of EPA to DHA in the phosphatidylcholine product represented by Formula (I) or the fatty acid composition is (about 0.40 to about 0.44):1. In certain embodiments, the area ratio of EPA to DHA in the phosphatidylcholine product represented by Formula (I) or the fatty acid composition is (about 0.42 to about 0.46):1. In certain embodiments, the area ratio of EPA to DHA in the phosphatidylcholine product represented by Formula (I) or the fatty acid composition is (about 0.44 to about 0.48):1. In certain embodiments, the area ratio of EPA to DHA in the phosphatidylcholine product represented by Formula (I) or the fatty acid composition is (about 0.46 to about 0.50):1. In certain embodiments, the area ratio of EPA to DHA in the phosphatidylcholine product represented by Formula (I) or the fatty acid composition is (about 0.48 to about 0.52):1. In certain embodiments, the area ratio of EPA to DHA in the phosphatidylcholine product represented by Formula (I) or the fatty acid composition is (about 0.50 to about 0.54):1. In certain embodiments, the area ratio of EPA to DHA in the phosphatidylcholine product represented by Formula (I) or the fatty acid composition is (about 0.52 to about 0.56):1. In certain embodiments, the area ratio of EPA to DHA in the phosphatidylcholine product represented by Formula (I) or the fatty acid composition is (about 0.54 to about 0.58):1. In certain embodiments, the area ratio of EPA to DHA in the phosphatidylcholine product represented by Formula (I) or the fatty acid composition is (about 0.56 to about 0.60):1. In certain embodiments, the area ratio of EPA to DHA in the phosphatidylcholine product represented by Formula (I) or the fatty acid composition is (about 0.44 to about 0.46):1. In certain embodiments, the area ratio of EPA to DHA in the phosphatidylcholine product represented by Formula (I) or the fatty acid composition is (about 0.43 to about 0.47):1. In certain embodiments, the area ratio of EPA to DHA in the phosphatidylcholine product represented by Formula (I) or the fatty acid composition is (about 0.42 to about 0.48):1. In certain embodiments, the area ratio of EPA to DHA in the phosphatidylcholine product represented by Formula (I) or the fatty acid composition is (about 0.41 to about 0.49):1. In certain embodiments, the area ratio of EPA to DHA in the phosphatidylcholine product represented by Formula (I) or the fatty acid composition is (about 0.40 to about 0.50):1. In certain embodiments, the area ratio of EPA to DHA in the phosphatidylcholine product represented by Formula (I) or the fatty acid composition is (about 0.39 to about 0.51):1. In certain embodiments, the area ratio of EPA to DHA in the phosphatidylcholine product represented by Formula (I) or the fatty acid composition is (about 0.38 to about 0.52):1. In certain embodiments, the area ratio of EPA to DHA in the phosphatidylcholine product represented by Formula (I) or the fatty acid composition is (about 0.35 to about 0.55):1. In certain embodiments, the area ratio of EPA to DHA in the phosphatidylcholine product represented by Formula (I) or the fatty acid composition is (about 0.35 to about 0.58):1. In certain embodiments, the area ratio of EPA to DHA in the phosphatidylcholine product represented by Formula (I) or the fatty acid composition is (about 0.33 to about 0.55):1. In certain embodiments, the area ratio of EPA to DHA in the phosphatidylcholine product represented by Formula (I) or the fatty acid composition is (about 0.37 to about 0.55):1. In certain embodiments, the area ratio of EPA to DHA in the phosphatidylcholine product represented by Formula (I) or the fatty acid composition is (about 0.39 to about 0.55):1. In some preferred embodiments, the area ratio of EPA to DHA in the phosphatidylcholine product represented by Formula (I) or the fatty acid composition is (about 0.38 to about 0.48):1 , more preferably (about 0.39 to about 0.47):1 .

In several embodiments, in the phosphatidylcholine product represented by Formula (l) or the fatty acid composition, an area percentage of a total amount of EPA and DHA to a total amount of fatty acids in the phosphatidylcholine product represented by Formula (I) is about 50% to about 95%, for example, about 55% to about 95%, about 55% to about 94%, about 55% to about 93%, about 55% to about 92%, about 55% to about 91%, about 55% to about 90%, about 55% to about 85%, about 60% to about 95%, about 65% to about 95%, about 65% to about 90%. In certain embodiments, in the phosphatidylcholine product represented by Formula (I) or the fatty acid composition, the area percentage of the total amount of EPA and DHA to the total amount of fatty acids in the phosphatidylcholine product represented by Formula (I) is about 65% to about 85%. In certain embodiments, in the phosphatidylcholine product represented by Formula (I) or the fatty acid composition, the area percentage of the total amount of EPA and DHA to the total amount of fatty acids in the phosphatidylcholine product represented by Formula (I) is about 66% to about 85%. In certain embodiments, in the phosphatidylcholine product represented by Formula (I) or the fatty acid composition, the area percentage of the total amount of EPA and DHA to the total amount of fatty acids in the phosphatidylcholine product represented by Formula (I) is about 67% to about 85%. In certain embodiments, in the phosphatidylcholine product represented by Formula (I) or the fatty acid composition, the area percentage of the total amount of EPA and DHA to the total amount of fatty acids in the phosphatidylcholine product represented by Formula (I) is about 68% to about 85%. In certain embodiments, in the phosphatidylcholine product represented by Formula (I) or the fatty acid composition, the area percentage of the total amount of EPA and DHA to the total amount of fatty acids in the phosphatidylcholine product represented by Formula (I) is about 65% to about 84%. In certain embodiments, in the phosphatidylcholine product represented by Formula (I) or the fatty acid composition, the area percentage of the total amount of EPA and DHA to the total amount of fatty acids in the phosphatidylcholine product represented by Formula (I) is about 67% to about 84%. In certain embodiments, in the phosphatidylcholine product represented by Formula (I) or the fatty acid composition, the area percentage of the total amount of EPA and DHA to the total amount of fatty acids in the phosphatidylcholine product represented by Formula (I) is about 68% to about 82%. In certain embodiments, in the phosphatidylcholine product represented by Formula (I) or the fatty acid composition, the area percentage of the total amount of EPA and DHA to the total amount of fatty acids in the phosphatidylcholine product represented by Formula (I) is about 70% to about 90%. In certain embodiments, in the phosphatidylcholine product represented by Formula (I) or the fatty acid composition, the area percentage of the total amount of EPA and DHA to the total amount of fatty acids in the phosphatidylcholine product represented by Formula (I) is about 75% to about 90%. In certain embodiments, in the phosphatidylcholine product represented by Formula (I) or the fatty acid composition, the area percentage of the total amount of EPA and DHA to the total amount of fatty acids in the phosphatidylcholine product represented by Formula (I) is about 75% to about 85%. In certain embodiments, in the phosphatidylcholine product represented by Formula (I) or the fatty acid composition, the area percentage of the total amount of EPA and DHA to the total amount of fatty acids in the phosphatidylcholine product represented by Formula (I) is about 77% to about 83%. In certain embodiments, in the phosphatidylcholine product represented by Formula (I) or the fatty acid composition, the area percentage of the total amount of EPA and DHA to the total amount of fatty acids in the phosphatidylcholine product represented by Formula (I) is about 79% to about 81%.

In several embodiments, in the phosphatidylcholine product represented by Formula (I) or the fatty acid composition, an area percentage of EPA to a total amount of fatty acids in the phosphatidylcholine product represented by Formula (I) is about 10% to about 40%, about 10% to about 39%, about 10% to about 38%, about 10% to about 37%, about 10% to about 36%, about 10% to about 35%, about 10% to about 34%, about 10% to about 33%, about 10% to about 32%, about 10% to about 31%, about 10% to about 30%, about 11% to about 40%, about 12% to about 40%, about 13% to about 40%, about 14% to about 40%, about 15% to about 40%, about 15% to about 39%, about 15% to about 38%, about 15% to about 37%, about 15% to about 36%, about 15% to about 35%, about 15% to about 34%, about 15% to about 33%, about 15% to about 32%, about 17% to about 33%, about 18% to about 32%, about 20% to about 30%, about 15% to about 30%, about 15% to about 28%, about 17% to about 28%, about 18% to about 28%; in certain embodiments, in the phosphatidylcholine product represented by Formula (I) or the fatty acid composition, the area percentage of EPA to the total amount of fatty acids in the phosphatidylcholine product represented by Formula (I) is about 20% to about 28%, about 20% to about 27%, about 20% to about 26%, about 20% to about 25%, about 20% to about 24%, about 20% to about 22%, about 21% to about 30%, about 21% to about 28%, about 21% to about 27%, about 21% to about 26%, about 21% to about 25%, about 21% to about 24%, about 21% to about 22%, about 22% to about 24%, about 22% to about 25%, about 22% to about 26%, about 22% to about 28%, about 22% to about 30%, about 23% to about 30%, about 23% to about 28%, about 23% to about 27%, about 23% to about 26%, about 23% to about 25%, about 24% to about 30%, about 24% to about 28%, about 24% to about 27%, about 24% to about 26%, about 25% to about 30%, about 25% to about 28%, about 25% to about 27%, about 26% to about 30%, about 26% to about 28%, about 27% to about 30%, about 27% to about 29%, about 28% to about 30%.

In several embodiments, in the phosphatidylcholine product represented by Formula (I) or the fatty acid composition, an area percentage of DHA to the total amount of fatty acids in the phosphatidylcholine product represented by Formula (I) is about 40% to about 70%, about 40% to about 69%, about 40% to about 68%, about 40% to about 67%, about 40% to about 66%, about 40% to about 65%, about 45% to about 65%, about 47% to about 63%, about 48% to about 62%, about 50% to about 60%. In certain embodiments, in the phosphatidylcholine product represented by Formula (I) or the fatty acid composition, the area percentage of DHA to the total amount of fatty acids in the phosphatidylcholine product represented by Formula (I) is about 50% to about 58%, about 50% to about 57%, about 50% to about 56%, about 50% to about 55%, about 50% to about 54%, about 51% to about 60%, about 51% to about 58%, about 51% to about 57%, about 51% to about 56%, about 51% to about 55%, about 51% to about 54%, about 52% to about 60%, about 52% to about 58%, about 52% to about 57%, about 52% to about 56%, about 52% to about 54%, about 53% to about 60%, about 53% to about 58%, about 53% to about 57%, about 53% to about 56%, about 53% to about 55%, about 54% to about 60%, about 54% to about 58%, about 54% to about 57%, about 54% to about 56%, about 55% to about 60%, about 55% to about 58%, about 55% to about 57%, about 56% to about 60%, about 56% to about 58%, about 57% to about 60%, about 58% to about 60%.

In certain embodiments, in the phosphatidylcholine product represented by Formula (I) or the fatty acid composition, the area percentage of EPA to the total amount of fatty acids in the phosphatidylcholine product represented by Formula (I) is about 10% to about 35%, for example, about 15% to about 32%, and the area percentage of DHA to the total amount of fatty acids in the phosphatidylcholine product represented by Formula (I) is about 40% to about 70%, for example about 40% to about 65%, for example, about 45% to about 65%; preferably, the area percentage of EPA to the total amount of fatty acids in the phosphatidylcholine product represented by Formula (I) is about 18% to about 32%, for example, about 18% to about 30%, for example, about 18% to 28%, and the area percentage of DHA to the total amount of fatty acids in the phosphatidylcholine product represented by Formula (I) is about 45% to about 65%, for example, about 50% to about 65%. For example, in the phosphatidylcholine product represented by Formula (I) or the fatty acid composition, the area percentage of EPA to the total amount of fatty acids in the phosphatidylcholine product represented by Formula (I) is about 15% to about 35%, and the area percentage of DHA to the total amount of fatty acids in the phosphatidylcholine product represented by Formula (I) is about 45% to about 65%. Alternatively, in the phosphatidylcholine product represented by Formula (I) or the fatty acid composition, the area percentage of EPA to the total amount of fatty acids in the phosphatidylcholine product represented by Formula (I) is about 15% to about 30%, and the area percentage of DHA to the total amount of fatty acid in the phosphatidylcholine product represented by Formula (I) is about 40% to about 65%. Alternatively, in the phosphatidylcholine product represented by Formula (I) or the fatty acid composition, the area percentage of EPA to the total amount of fatty acids in the phosphatidylcholine product represented by Formula (I) is about 15% to about 35%, and the area percentage of DHA to the total amount of fatty acids in the phosphatidylcholine product represented by Formula (I) is about 40% to about 65%.

In all embodiments, in addition to EPA and DHA, the fatty acids in the phosphatidylcholine product represented by Formula (I) or the fatty acid composition may also comprise the following n-3 fatty acids: DPA, heneicosapentaenoic acid (n-3), eicosatetraenoic acid (n-3), octadecatetraenoic acid (n-3), octadecatrienoic acid (n-3), etc.

In several embodiments, the fatty acids in the phosphatidylcholine product represented by Formula (I) or the fatty acid composition comprise EPA, DHA and DPA. In certain embodiments, in the phosphatidylcholine product represented by Formula (I) or the fatty acid composition, the area percentage of DPA to the total amount of fatty acids in the phosphatidylcholine product represented by Formula (I) is not more than 15%, not more than 12%, not more than 10%. In certain embodiments, in the phosphatidylcholine product represented by Formula (I) or the fatty acid composition, the area percentage of DPA to the total amount of fatty acids in the phosphatidylcholine product represented by Formula (I) is about 2% to about 15%, about 2% to about 12%, about 5% to about 15%, about 5% to about 13%, about 5% to about 12%, about 5% to about 10%, about 5% to about 9%, about 5% to about 8%, about 5% to about 7%, about 6% to about 8%, about 6% to about 10%, about 6% to about 12%, about 6% to about 15%, about 7% to about 15%, about 7% to about 13%, about 7% to about 11%, about 7% to about 10%, about 8% to about 15%, about 8% to about 13%, about 8% to about 11%, about 8% to about 10%, about 9% to about 15%, about 9% to about 13%, about 9% to about 11%, about 10% to about 15%, about 10% to about 14%, about 11% to about 15%, about 11% to about 13%.

In several embodiments, in the phosphatidylcholine product represented by Formula (I) or the fatty acid composition, an area percentage of eicosatetraenoic acid (n-3) to the total amount of fatty acids in the phosphatidylcholine product represented by Formula (I) is not more than 5%, not more than 3%, not more than 2.5%, not more than 2%, not more than 1.5%, not more than 1%, not more than 0.5%. In several embodiments, in the phosphatidylcholine product represented by Formula (I) or the fatty acid composition, the area percentage of eicosatetraenoic acid (n-3) to the total amount of fatty acids in the phosphatidylcholine product represented by Formula (I) is about 0.01% to about 5%, about 0.01% to about 3%, about 0.2% to about 4%, about 0.5% to about 4%, about 0.5% to about 2%.

In several embodiments, in the phosphatidylcholine product represented by Formula (I) or the fatty acid composition, an area percentage of octadecatetraenoic acid (n-3) to the total amount of fatty acids in the phosphatidylcholine product represented by Formula (I) is not more than 5%, not more than 3%, not more than 2%, not more than 1.5%, not more than 1%, not more than 0.5%.

In several embodiments, in the phosphatidylcholine product represented by Formula (I) or the fatty acid composition, an area percentage of heneicosapentaenoic acid (n-3) to the total amount of fatty acids in the phosphatidylcholine product represented by Formula (I) is not more than 5%, not more than 3%, not more than 2%, not more than 1.5%, not more than 1 %, not more than 0.5%, or is about 1.5% to about 5%, about 1.5% to about 4%, about 2% to about 5%, about 2% to about 4%, about 2% to about 3%.

In several embodiments, in the phosphatidylcholine product represented by Formula (I) or the fatty acid composition, an area percentage of octadecatrienoic acid (n-3) to the total amount of fatty acids in the phosphatidylcholine product represented by Formula (I) is not more than 2%, not more than 1.5%, not more than 1 %, not more than 0.9%, not more than 0.7%.

In various embodiments, in addition to EPA and DHA, the fatty acids in the phosphatidylcholine product represented by Formula (I) or the fatty acid composition may also comprise the following n-6 fatty acids: octadecadienoic acid (n-6), octadecatrienoic acid (n-6), eicosatrienoic acid (n-6), eicosatetraenoic acid (n-6), docosapentaenoic acid (n-6), etc.

In several embodiments, in the phosphatidylcholine product represented by Formula (I) or the fatty acid composition, an area percentage of eicosatetraenoic acid (n-6) to the total amount of fatty acids in the phosphatidylcholine product represented by Formula (I) is not more than 5%, not more than 3%, not more than 2%, not more than 1.5%, not more than 1 %, not more than 0.5%. In several embodiments, in the phosphatidylcholine product represented by Formula (I) or the fatty acid composition, the area percentage of eicosatetraenoic acid (n-6) to the total amount of fatty acids in the phosphatidylcholine product represented by Formula (I) is about 0.01% to about 5%, about 0.01% to about 3%, about 0.2% to about 4%, about 0.5% to about 4%, about 0.5% to about 2%.

In several embodiments, in the phosphatidylcholine product represented by Formula (I) or the fatty acid composition, an area percentage of docosapentaenoic acid (n-6) to the total amount of fatty acids in the phosphatidylcholine product represented by Formula (I) is not more than 5%, not more than 3%, not more than 2%, not more than 1.5%, not more than 1%, not more than 0.5%, or is about 0.1% to about 5%, about 0.5% to about 5%, about 1% to about 5%, about 1.2% to about 5%, about 1.5% to about 5%, about 2% to about 5%, about 2% to about 4%.

In several embodiments, in the phosphatidylcholine product represented by Formula (I) or the fatty acid composition, an area percentage of a total amount of n-6 fatty acids to the total amount of fatty acids in the phosphatidylcholine product represented by Formula (I) is not more than 10%, not more than 8%, not more than 7%, not more than 5%, not more than 4%. In several embodiments, in the phosphatidylcholine product represented by Formula (I) or the fatty acid composition, the area percentage of the total amount of n-6 fatty acids to the total amount of fatty acids in the phosphatidylcholine product represented by Formula (I) is about 0.5% to about 10%, about 1% to about 5%, about 1.5% to about 5%, about 2% to about 5%.

In certain embodiments, in the phosphatidylcholine product represented by Formula (I) or the fatty acid composition, an area percentage of n-3 fatty acids to the total amount of fatty acids in the phosphatidylcholine product represented by Formula (I) is about 60% or more, for example about 65% or more. In various embodiments, in the phosphatidylcholine product represented by Formula (I) or the fatty acid composition, the area percentage of the total amount of n-3 fatty acids to the total amount of fatty acids in the phosphatidylcholine product represented by Formula (I) is not less than 70%, not less than 75%, not less than 80%, not less than 85%, not less than 90%, not less than 91%, not less than 92%, not less than 93%, not less than 95%. In various embodiments, in the phosphatidylcholine product represented by Formula (I) or the fatty acid composition, the area percentage of the total amount of n-3 fatty acids to the total amount of fatty acids in the phosphatidylcholine product represented by Formula (I) is about 65% to about 99%, about 65% to about 96%, about 75% to about 95%. In various embodiments, in the phosphatidylcholine product represented by Formula (I) or the fatty acid composition, the area percentage of the total amount of n-3 fatty acids to the total amount of fatty acids in the phosphatidylcholine product represented by Formula (I) is about 80% to about 95%, about 82% to about 95%, about 85% to about 95%.

In several embodiments, in the phosphatidylcholine product represented by Formula (I) or the fatty acid composition, the area percentage of the total amount of n-3 and n-6 fatty acids to the total amount of fatty acids in the phosphatidylcholine product represented by Formula (I) is not less than 80%, not less than 85%, not less than 90%, not less than 92%, not less than 95%, not less than 96%, not less than 97%, not less than 98%. In several embodiments, in the phosphatidylcholine product represented by Formula (I) or the fatty acid composition, the area percentage of the total amount of n-3 and n-6 fatty acids to the total amount of fatty acids in the phosphatidylcholine product represented by Formula (I) is about 80% to about 99%, about 85% to about 99%.

In several embodiments, in the phosphatidylcholine product represented by Formula (I) or the fatty acid composition, the area percentage of the total amount of other polyunsaturated fatty acids except n-3 and n-6 fatty acids to the total amount of fatty acids in the phosphatidylcholine product represented by Formula (I) is not more than 5%, not more than 3%, not more than 2%, not more than 1%, not more than 0.5%.

In certain embodiments, in the phosphatidylcholine product represented by Formula (I) or the fatty acid composition, the area percentage of saturated fatty acids to the total amount of fatty acids in the phosphatidylcholine product represented by Formula (I) is not more than 20%, not more than 5%, not more than 3%, not more than 2%, not more than 0.5%, not more than 0.3%, not more than 0.2%. In certain embodiments, in the phosphatidylcholine product represented by Formula (I) or the fatty acid composition, the area percentage of saturated fatty acids to the total amount of fatty acids in the phosphatidylcholine product represented by formula (I) is about 0.01 % to about 5%, about 0.05% to about 3%.

In several embodiments, in the phosphatidylcholine product represented by Formula (I) or the fatty acid composition, an area percentage of C16:0 to a total amount of fatty acids in the phosphatidylcholine product represented by Formula (I) is not more than 5%, not more than 3%, not more than 2%, not more than 0.5%, not more than 0.3%, not more than 0.2%.

In certain embodiments, in the phosphatidylcholine product represented by Formula (I) or the fatty acid composition, an area percentage of monounsaturated fatty acids to the total amount of fatty acids in the phosphatidylcholine product represented by Formula (I) is not more than 10%, not more than 8%, not more than 5%, not more than 3%, not more than 2%, not more than 0.5%, not more than 0.3%, not more than 0.2%. In certain embodiments, in the phosphatidylcholine product represented by Formula (I) or the fatty acid composition, the area percentage of monounsaturated fatty acids to the total amount of fatty acids in the phosphatidylcholine product represented by Formula (I) is about 0.01% to about 6%, about 0.05% to about 6%.

In several embodiments, in the phosphatidylcholine product represented by Formula (I) or the fatty acid composition, an area percentage of C18:1 n-9 to the total amount of fatty acids in the phosphatidylcholine product represented by Formula (I) is not more than 5%, not more than 3%, not more than 2%, not more than 0.5%, not more than 0.3%, not more than 0.2%.

In certain embodiments, in the phosphatidylcholine product represented by Formula (I) or the fatty acid composition, an area percentage of a total amount of saturated fatty acids and monounsaturated fatty acids to a total amount of fatty acids in the phosphatidylcholine product represented by Formula (I) is not more than 15%, not more than 10%, not more than 5%, not more than 1%, not more than 0.8%, not more than 0.6%, not more than 0.5%.

In certain embodiments, in the phosphatidylcholine product represented by Formula (I) or the fatty acid composition, an area percentage of n-3 fatty acids to a total amount of fatty acids in the phosphatidylcholine product represented by Formula (I) is about 60% or more, for example, about 65% or more, preferably about 65% to about 99%, more preferably about 65% to about 96%, most preferably about 85% to about 95%.

In certain embodiments, in the phosphatidylcholine product represented by Formula (I) or the fatty acid composition, relative to n-3 fatty acids, an area percentage of EPA is about 15% to about 40%, preferably about 20% to about 30%, more preferably about 21% to about 30%, for example, 23% to 27%; and/or relative to n-3 fatty acids, an area percentage of DHA is about 45% to about 75%, for example, about 50% to about 75%, preferably about 55% to about 70%, more preferably about 57% to about 61%.

In certain embodiments, in the phosphatidylcholine product represented by Formula (I) or the fatty acid composition, relative to n-3 fatty acids, an area percentage of DPA is about 2% to about 20%, for example, about 2% to about 15%, about 2% to about 14%, about 2% to about 13%, about 2.5% to about 15%, about 2.5% to about 14%, about 2.5% to about 13%, about 3% to about 15%, about 4% to about 15%, about 5% to about 15%, about 6% to about 15%, about 7% to about 15%, about 7% to about 14%, about 7% to about 13%.

Unless otherwise indicated, the term "fatty acid composition" herein, when it is not used in conjunction with "free acid form" or "free acid type", "ethyl ester form" or "ethyl ester type", means a composition comprising the phosphatidylcholine product represented by Formula (I), which is obtained by purifying after a reaction of free fatty acid with glycerol phosphatidylcholine, and can also be referred to as a "phospholipid composition". When a content of the phosphatidylcholine product in the fatty acid composition is 90% or more in weight percentage, the fatty acid composition may also be referred to as a phosphatidylcholine product.

In all embodiments, a content of the phosphatidylcholine product represented by Formula (I) in the fatty acid composition is about 50% or more. In certain embodiments, the content of the phosphatidylcholine product represented by Formula (I) in the composition is about 50% or more, about 60% or more, about 70% or more, about 80% or more, about 90%, about 91% or more, about 92% or more, about 93% or more, about 94% or more, about 95% or more, about 96% or more, about 97% or more, about 98% or more, about 99% or more.

In all embodiments, the fatty acid composition herein comprises almost no phosphatidylethanolamine (PE), phosphatidylinositol (PI) or phosphatidylserine (PS). In certain embodiments, the fatty acid composition herein does not comprise phosphatidylethanolamine (PE), phosphatidylinositol (PI), or phosphatidylserine (PS).

Unless otherwise indicated, the expression "comprise almost no" herein means that the weight percentage content of the object defined by this term relative to the total weight is 1% or less, for example, 0.5% or less, preferably 0.1% or less.

Further, in certain embodiments, the phosphatidylcholine product is obtained by a reaction of a fatty acid composition in free acid form. In certain embodiments, the phosphatidylcholine product is obtained by a condensation reaction of a fatty acid composition in free acid form. In certain embodiments, the phosphatidylcholine product is obtained by the condensation reaction of a fatty acid composition in free acid form with glycerol phosphatidylcholine (GPC). In certain embodiments, the phosphatidylcholine product is obtained by the condensation reaction of a fatty acid composition in free acid form with glycerol phosphatidylcholine (GPC) in the presence of alkali and a condensation agent. In certain embodiments, a reaction solvent for the condensation reaction is one or more selected from dichloromethane, chloroform, diethylformamide, tetrahydrofuran, N,N-dimethylformamide, or dioxane. In certain embodiments, the alkali for the condensation reaction is one or more selected from sodium carbonate, potassium carbonate, potassium acetate, triethylamine, 4-dimethylaminopyridine or N,N-diisopropylethylamine. In certain embodiments, the condensation agent is one or more selected from 2-(7-azabenzotriazole)-N, N, N', N'-tetramethyluronium hexafluorophosphate, benzotriazole-N,N,N',N'-tetramethyluronium hexafluorophosphate or N,N'-dicyclohexylcarbodiimide.

In all embodiments, the components and contents of fatty acids in the fatty acid composition in free acid form as an intermediate are the same as the definitions for components and contents of fatty acids in the phosphatidylcholine product represented by Formula (I) above. For example, in several embodiments, an area ratio of EPA to DHA in the fatty acid composition in free acid form is (about 0.33 to about 0.6):1, or the various ratios described above; in several embodiments, in the fatty acid composition in free acid form, an area percentage of a total amount of EPA and DHA to a total amount of fatty acids is about 65% to about 90%, or the various ranges described above; in several embodiments, in the fatty acid composition in free acid form, an area percentage of EPA to a total amount of fatty acids is about 15% to about 35%, or the various ranges described above; in several embodiments, in the fatty acid composition in free acid form, an area percentage of DHA to a total amount of fatty acids is about 45% to about 65%, or the various ranges described above; in certain embodiments, in the fatty acid composition in free acid form, an area percentage of DPA to a total amount of fatty acids is not more than 15%, or the various ranges mentioned above.

The main component of the fatty acid composition provided herein is phosphatidylcholine product, which mainly comprises polyunsaturated fatty acids, and the most important components thereof are DHA and EPA, and the increase of the contents of monounsaturated fatty acids or/and saturated fatty acids is unfavorable to the pharmacodynamic effect of the fatty acid composition. The applicant creatively finds that, compared to the model group, the composition provided herein has a significant statistical difference in the efficacy on IBD mice, which is better than the first-line therapeutic medicament sulfasalazine.

The fatty acid composition provided herein may be used for the prevention or treatment of inflammatory bowel disease. Inflammatory bowel disease (IBD) herein comprises Ulcerative colitis (UC) and Crohn's disease (CD).

"EPA residue(s)", "DHA residue(s)" or other "fatty acid residues" mentioned herein are products of fatty acid dehydroxylation after an esterification reaction of the corresponding fatty acids with two free hydroxyl groups in a glycerol phosphatidylcholine molecule.

The area percentage of fatty acids mentioned herein, such as "area percentage of EPA to a total amount of fatty acids in the composition", etc., which is one of the manners to characterize content that can be understood in the art, is the calculated percentage of a chromatographic peak area of each fatty acid methyl ester to a chromatographic peak area of total fatty acid methyl ester, after the phosphatidylcholine product of Formula (I) provided herein is esterified into fatty acid methyl esters.

"Area ratio between EPA and DHA" mentioned herein is a ratio of the area percentage of EPA methyl ester to the area percentage of DHA methyl ester after the phosphatidylcholine product of Formula (I) provided herein is esterified into fatty acid methyl ester.

When "about" mentioned herein is used with a cited value, it means the cited value, and comprises the range of +5% or -5%, preferably the range of ±2% or ±1% of the value. For example, about 50% means 50% and +5% or -5% of 50%, that is, 45% to 55%.

Unless otherwise indicated, the term "saturated fatty acid" herein refers to fatty acids that do not comprise unsaturated bond(s), and the term "unsaturated fatty acid" refers to fatty acids comprising one or more carbon-carbon double bonds, comprising monounsaturated fatty acids and polyunsaturated fatty acids.

Unless otherwise indicated, singular terms encompass plural terms, and plural terms encompass singular terms herein.

In some embodiments, the application relates to a pharmaceutical preparation, comprising the above-mentioned fatty acid composition and pharmaceutically acceptable excipients.

This application can prepare pharmaceutical preparations for use, such as tablets, pills, capsules, powders, granules, ointments, emulsions, suspensions, suppositories, injections, gels, etc., by combining the fatty acid composition disclosed in this application with suitable pharmaceutically acceptable excipients. Typical routes for administering the fatty acid composition of the application include, but are not limited to, oral, rectal, topical, parenteral, intraperitoneal, intramuscular, subcutaneous, and intravenous administration. The excipients described herein may be any pharmaceutically acceptable excipients, such as, but not limited to, solvents, propellants, solubilizers, cosolvents, emulsifiers, colorants, disintegrants, fillers, lubricants, wetting agents, osmotic pressure regulators, stabilizers, glidants, flavoring agents, preservatives, suspending agents, antioxidants, penetration enhancers, pH regulators, surfactants, diluents, etc. For other available pharmaceutically acceptable pharmaceutical excipients, they can be found, for example, in "Handbook of Pharmaceutical Excipients" (Fourth Edition), edited by R.C. Rowe et al., translated by Zemin ZHENG, 2005, Chemical Industry Press.

The pharmaceutical preparations of the application may be manufactured by methods well known in the art, such as conventional mixing method, dissolution method, granulation method, sugar-coated pill preparation method, grinding method, emulsification method, lyophilization method, etc.

In some embodiments, the pharmaceutical formulation is in oral form. For oral administration, the pharmaceutical preparation may be formulated by mixing the above fatty acid composition with pharmaceutically acceptable excipients well known in the art.

As well known by the skilled in the art, the administration dosage of a medicament depends on a variety of factors, comprising, but not limited to, the following factors: activity of the specific compound used, patient's age, patient's body weight, patient's health condition, patient's diet, time of administration, administration manner, etc.; the daily administration dosage of the fatty acid composition herein may be verified according to traditional treatment regimens. An exemplary effective dosage range is about 100 mg to about 20,000 mg per day, and an exemplary administration manner is to administer the fatty acid composition herein in a single daily dose, or the total daily dose may be divided into two, three, or four doses per day.

Unless otherwise indicated, the terms "patient", "subject" and "individual" herein are used interchangeably and refer to human or non-human animals (e.g., primates, rodents, etc.).

Unless otherwise indicated, the term "ester-type fish oil" herein refers to fish oil that obtained by purifying from raw materials such as fish and algae, in which fatty acids form ester groups, and mainly includes ethyl ester-type fish oil and triglyceride-type fish oil.

In some embodiments, the application relates to use of a phosphatidylcholine product represented by Formula (I) or a fatty acid composition comprising the same in manufacturing a medicament for preventing or treating inflammatory bowel disease.

In some embodiments, the application relates to a phosphatidylcholine product represented by Formula (I) or a fatty acid composition comprising the same for use in preventing or treating inflammatory bowel disease.

In some embodiments, the application relates to a method for preventing or treating inflammatory bowel disease, comprising administering to patient a therapeutically effective amount of a phosphatidylcholine product represented by Formula (I) or a fatty acid composition comprising the same.

In some preferred embodiments, said inflammatory bowel disease is selected from ulcerative colitis or Crohn's disease.

In some embodiments, the application relates to a method for preparing a fatty acid composition comprising a phosphatidylcholine product represented by Formula (I), comprising subjecting free fatty acids to a condensation reaction with glycerol phosphatidylcholine; and purifying resulted reaction product to obtain the fatty acid composition.

In some preferred embodiments, the condensation reaction is performed in the presence of alkali, reaction solvents and condensation agents. In some preferred embodiments, the reaction solvent is one or more selected from dichloromethane, chloroform, diethylformamide, tetrahydrofuran, N,N-dimethylformamide or dioxane. In some preferred embodiments, the alkali is one or more selected from sodium carbonate, potassium carbonate, potassium acetate, triethylamine, 4-dimethylaminopyridine or N,N-diisopropylethylamine. In some preferred embodiments, the condensation agent is one or more selected from 2-(7-azabenzotriazole)-N, N, N', N'-tetramethyluronium hexafluorophosphate, benzotriazole-N,N,N',N'-tetramethyluronium hexafluorophosphate, carbodiimide, and the carbodiimide is, for example, selected from dicyclohexylcarbodiimide (DCC), diisopropylcarbodiimide (DIC), 1-(3-dimethylaminopropyl)-3-ethylcarbodiimide (EDCI).

In some preferred embodiments, the condensation reaction is performed under vacuum condition at room temperature. In some preferred embodiments, the purifying in the second step may be selected from extraction, washing, filtration or HPLC purification.

In some preferred embodiments, the above-mentioned method further comprises: hydrolyzing an ester-type fish oil to obtain free fatty acids. In a further preferred embodiment, a crude triglyceride oil extracted from fish and algae is refined (such as alkali refining) and purified (such as urea inclusion, thin film evaporation, molecular distillation, etc.) to obtain the ester-type fish oil. In other preferred embodiments, the ester-type fish oil is hydrolyzed by a saponification reaction. For example, the ester-type fish oil is preferably hydrolyzed through the following process: the ester-type fish oil is heated to a temperature of 30°C or higher; sodium hydroxide is dissolved in water, to which ethanol is added to obtain a sodium hydroxide-ethanol solution; the sodium hydroxide-ethanol solution is added into the heated ester-type fish oil for reaction; n-heptane is added into the reaction solution and the pH of the reaction solution is adjusted with hydrochloric acid; the reaction product is left to stand for separation, followed by drying and filtration, and the dried reaction product is concentrated under reduced pressure to obtain the free fatty acid.

Unless otherwise indicated, the term "room temperature" herein refers to a temperature of about 20 to 35°C.

Unless otherwise indicated, the terms "comprise", "include" and "contain" or equivalents herein, which is an open-ended mode expression, means that in addition to the listed elements, components and steps, other unspecified elements, components and steps may also be covered.

For the purpose of describing and disclosing, all patents, patent applications and other established publications are expressly incorporated herein by reference. These publications are provided solely for their disclosure prior to the filing date of this application. All statements regarding the dates of these documents or the representation of the contents of these documents are based on the information available to the applicants and do not constitute any admission as to the correctness of the dates of these documents or the contents of these documents. Moreover, any reference to these publications herein does not constitute an admission that the publications form part of the common general knowledge in the art in any country.

There are no special limitations to the preparation method of the fatty acid composition herein, which may be prepared according to the following route:
1. Using fatty acid and glycerol phosphatidylcholine as raw materials to obtain a fatty acid composition after reaction and purification;
2. Using ester-type fish oil as a raw material, firstly hydrolyzing it to obtain free fatty acids, which are then reacted with glycerol phosphatidylcholine and purified to obtain a fatty acid composition;
3. Using fish, algae, etc. as raw materials to extract crude triglyceride oil, and obtaining ester-type fish oil after refining and purification, and then hydrolyzing it to obtain free fatty acids, which are then reacted with glycerol phosphatidylcholine and purified to obtain a fatty acid composition.

An exemplary preparation method of the composition provided herein is as follows:

### (1) Preparation of fish oil composition in ethyl ester form

The fish oil composition in ethyl ester form with different kinds and contents of fatty acids may be prepared by using preparation methods well known in the art.

An exemplary preparation method comprises the following steps: extracting crude triglyceride oil from fish (anchovy, tuna, sardine, mackerel, and herring); then, subjecting the crude triglyceride oil to alkali refining, deodorization, urea inclusion, thin film evaporation, and molecular distillation. For example, the process parameters in the molecular distillation step are adjusted to intercept fish oil with different ratios of fatty acids, or the fish oil composition with the corresponding composition in ethyl ester form is directly purchased, or almost pure fatty acids are used for mixing, or the commercially available fish oils in ethyl ester form are used for mixing. Commercially available fish oils in ethyl ester form may be purchased from, for example, Zhoushan Sinomega Biotech Engineering Co., Ltd., Deyang Huatai Biopharm Resource Co., Ltd. etc.

### (2) Preparation of fatty acid composition in free acid form:

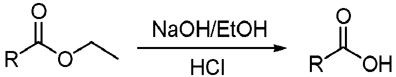

The fatty acid composition in free acid form may be prepared from the fatty acid composition in ethyl ester form (also referred to as "ethyl ester-type fish oil") by using a saponification reaction well known in the art. An exemplary preparation method is as follows:
A certain amount of fatty acid ethyl ester is weighed, added into a flask, and heated to 30°C. Sodium hydroxide is weighed 0.15 times the weight of the fatty acid and dissolved in an appropriate amount of purified water, to which an appropriate amount of ethanol is added to obtain a sodium hydroxide-ethanol solution. The above sodium hydroxide-ethanol solution is added dropwise to the flask with the fatty acid ethyl ester, stirred rapidly, and reacted for 1-2 hours. n-heptane is added to the reaction solution, the pH of which is adjusted to 1-2 with 3N hydrochloric acid. After standing for separation, n-heptane is washed once with purified water, dried with anhydrous sodium sulfate, filtered, and the filtrate is concentrated under reduced pressure to solvent-free to obtain free fatty acids.

### (3) Preparation of fish oil composition in phosphatidylcholine form

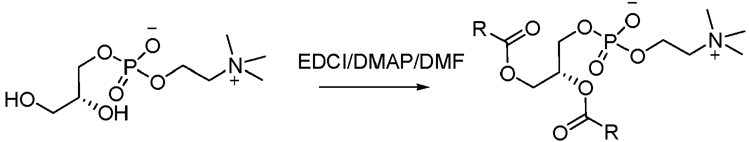

The fish oil composition in phosphatidylcholine form is prepared by a method for preparing phospholipids well known in the art, such as CN105753897A, which is incorporated herein by reference in its entirety.

The exemplary preparation method comprises the following steps: adding GPC, EDCI, DMAP, the free fatty acid obtained in step (2) and solvent DMF into a reaction vessel, stirring, vacuum pumping, with N₂/Ar constant pressure, and then stirring the same at room temperature to react completely, and the end point of the reaction may be judged by whether the reaction solution is clear. The posttreatment may use conventional purification methods in the art. Exemplary purification methods are, such as, extraction, including the means which can extract the desired product from the system, for example, extraction, back extraction, supercritical fluid extraction, etc.; washing, including the means which can keep the pH value of the system neutral, for example, acid washing, alkaline washing, water washing, salt washing and the like; filtration, including the means which separate solid and liquid substances in the system, for example, atmospheric filtration, atmospheric suction filtration, vacuum filtration and the like; and HPLC purification, etc., which can be referred to, for example, CN110054565A, CN105873893A, CN102285880A, etc., which is incorporated herein by reference in its entirety.

Among the above-mentioned exemplary preparation methods, conventional preparation methods in the art may maturely prepare ethyl ester-type fatty acid compositions (also called "ethyl ester-type fish oil") with different fatty acid component and content. Each fatty acid component and content of the fatty acid compositions in free acid form, which are obtained after the complete reaction of step (2) with these ethyl ester-type fatty acid compositions having different component and content, are not significantly different from those in the ethyl ester-type fatty acid composition; the fatty acid component and content in the phosphatidylcholine product, which is obtained after the complete reaction of step (3), also do not change significantly.

After obtaining the fatty acid composition, the following methods are used herein to determine the content of the phosphatidylcholine product and the kinds and contents of the fatty acids in the phosphatidylcholine product:

### 1. Method for determining the content of phosphatidylcholine product

1.1 Content measurement: Performing the experiment according to high performance liquid chromatography (Pharmacopoeia of the People's Republic of China, 2015 Edition, Volume IV, General Rule 0512).

Chromatographic conditions and system suitability test: using diol-silica as filler, using n-hexane-isopropanol-methanol-water-glacial acetic acid-triethylamine (10:60:25:10:0.5:0.05) as mobile phase A, and using n-hexane-isopropanol-glacial acetic acid-triethylamine (45:55:0.5:0.05) as mobile phase B; performing gradient elution according to the following table; using an evaporative light scattering detector as detector (reference conditions: the temperature of the drift tube is 50°C, and the flow rate of the carrier gas is 2.0L/min).

| time | A% | B% |
|---|---|---|
| 0 | 5 | 95 |
| 10 | 50 | 50 |
| 15 | 100 | 0 |
| 23 | 100 | 0 |
| 24 | 5 | 95 |
| 30 | Stop | |

An appropriate amount of phosphatidylcholine reference substance (EPA/DHA-PC) is taken and dissolved in methanol to prepare mixed solutions respectively comprising 40µg, 100µg, 500µg, 1000µg, and 2000µg of the above reference substance per 1mL. 5µL of each of the above solutions is taken and injected into the liquid chromatograph for test, and the number of theoretical plates should not be less than 1500 in terms of phosphatidylcholine.

Measurement method: An appropriate amount of the product is taken, precisely weighed, dissolved and diluted with methanol to prepare a solution comprising 1.5 mg of the product per 1 mL, which is shaken up, and used as the test sample. 5µL of each of the above sample solutions is precisely measured and injected into the liquid chromatograph, the chromatogram is recorded, and the regression equation is calculated with the log value of the concentration of the reference substance solution and the log value of the corresponding peak area. The content of phosphatidylcholine is calculated using the regression equation.

1.2 Related substances: An appropriate amount of the product is taken, precisely weighed, dissolved and diluted with methanol to prepare a solution comprising 30 mg of the product per 1 mL as the test solution. According to the chromatographic conditions under the item of content measurement, 5µL of the test solution is taken and injected into the liquid chromatograph, and the content of related substances is calculated with the regression equation under the item of content measurement.

### 2. Method for measuring fatty acid composition

(1) Sample processing: 0.5g of the test sample is taken, precisely weighed, and placed in a 10mL volumetric flask. Sample preparation and processing is performed by referring to the methyl esterification method for measuring fatty acid composition under USP41 <401> Fat and Fixed Oil "OMEGA-3 FATTY ACIDS DETERMINATION AND PROFILE". And the test sample solution is prepared.

### (2) Determination of fatty acid component

Chromatographic conditions and system suitability test: a capillary column with polyethylene glycol as the stationary liquid is used (for example, Agilent CP-Wax 52 CB 50mx0.25mm, 0.2µm); the initial temperature of the temperature programming is 180°C, and the temperature is raised to 220°C at 1.5°C/min, then to 240°C at 2°C/min, and maintained for 15 minutes; the temperature of the injection port is 250°C; the temperature of the detector is 270°C; the carrier gas is helium, and the flow rate is 1.5mL per minute; the injection volume is 1µL, and the split flow ratio is 20:1.

System suitability solution: referring to USP41 <401> Fat and Fixed Oil "OMEGA-3 FATTY ACIDS DETERMINATION AND PROFILE" system suitability solution preparation and requirements.

Positioning solution 1: 0.05g of USP "Fish Oil" reference solution is taken, precisely weighed, placed in a 10mL volumetric flask, dissolved and diluted to the mark with anti-oxidant solution, which is shaken up to obtain the stock solution of positioning solution 1. 2.0 mL of the stock solution of positioning solution 1 is taken, and methylated the same as the test sample. The methyl ester solution was directly used as the positioning solution 1 without dilution (the reference sample has its standard chromatogram);

Positioning solution 2: about 10mg of each of methyl cis-6,9,12-octadecatrienoate (C18:3 n-6), methyl cis-11,14-eicosadienoate (C20:2 n-6), methyl cis-8,11,14-eicosatrienoate (C20:3 n-6), methyl cis-11,14,17-eicosatrienoate (C20:3 n-3) and methyl cis-4,7,10,13,16-docosapentaenoate (C22:5 n-6) etc. are taken, precisely weighed, and placed in a same 20mL volumetric flask, dissolved and diluted to the mark with anti-oxidant solution, which is shaken up to obtain the solution (0.5mg/mL).

Determination method: 1µL of the positioning solutions and the test sample solution are precisely measured, injected into the gas chromatograph, respectively, and the chromatogram was recorded. The content of each positioned fatty acid, the area ratio between EPA and DHA, total n-3 content, total n-6 content, contents of other polyunsaturated fatty acids, saturated fatty acids, monounsaturated fatty acids and undetermined fatty acids etc. are calculated with the area normalization method. The experimental results show that the fatty acid composition provided herein may improve the damage of the colon tissue in the IBD model animal to a certain extent, and has a certain anti-IBD activity, which is superior to the medicament sulfasalazine, superior to the phosphatidylcholine products within the scope of claim 1 of patent application WO2014154683, such as the phosphatidylcholine product in Example 1a thereof, the commercially available LIPOID S-100 type phosphatidylcholine product or other commercially available high-purity phosphatidylcholine products. The experiments have also found that the activity of the phosphatidylcholine product herein is superior to that of free acid-type fatty acid composition with similar fatty acid ratio and content. Further, compared with triglyceride-type fatty acid composition with similar fatty acid ratio and content, DAI comprehensive index inhibition rate has increased to 150% or more; and compared with the ethyl ester-type fatty acid composition with similar fatty acid ratio and content, the histopathological score has increased to 116% or more, wherein the triglyceride-type, free acid-type or ethyl ester-type fatty acid composition with the similar fatty acid ratio and content is the triglyceride type raw material, the free acid type intermediate or the ethyl ester type raw material of the previous step in the synthesis process of the corresponding phosphatidylcholine type fish oil.

### EMBODIMENTS OF THE INVENTION

The technical solutions in the examples of the application will be described clearly and completely below. Obviously, the described examples are only a part of the examples of the application, not all of the examples. Based on the examples in the application, all other examples obtained by those skilled in the art without creative work shall fall within the protection scope of the application.

Unless otherwise indicated, the reagents, materials, and devices used in the following examples are all commercially available reagents, materials, and devices.

### Examples 1 to 9

Using commercially available ethyl ester-type fish oils with different fatty acid contents as raw materials, the fatty acid composition was prepared according to the following method:
(1) The ethyl ester-type fish oils with different compositions of fatty acids were heated to 30°C, to which a solution of sodium hydroxide, ethanol and water (wherein, sodium hydroxide was weighed and dissolved in purified water, and 95% ethanol was added thereto to obtain the solution) was added dropwise. It was stirred quickly to react for 1.5h; n-heptane with a weight ratio of 1:1 to ethyl ester-type fish oil was added into the reaction solution, and the pH value of the reaction solution was adjusted to 1.5 with 3N hydrochloric acid. It was stood for separation, and washed with water to pH 6-7, dried with anhydrous sodium sulfate and filtered, and the obtained filtrate was concentrated under reduced pressure to solvent-free to obtain free fatty acids; wherein the weight ratio among the ethyl ester-type fish oil, sodium hydroxide and ethanol was 1:0.15:0.75;
(2) After mixing and dissolving glycerol phosphatidylcholine (GPC), EDCI, 4-dimethylaminopyridine (DMAP), the free fatty acid obtained in step (1) and the solvent N,N-dimethylformamide (DMF), it was vacuumed (wherein, the molar ratio among GPC, EDCI, DMAP and free fatty acid was 1.0: 2.86: 0.3: 2.6), and stirred under N₂/Ar constant pressure at room temperature to complete the reaction. HPLC purification was performed by using C18 column with 93% methanol/water as the mobile phase, the target components were collected, and the fatty acid composition was obtained after vacuum concentration, wherein the phosphatidylcholine product represented by Formula (I) was included.

The contents of the phosphatidylcholine products in the obtained fatty acid compositions were determined by the method described above, and the contents of the phosphatidylcholine products (in wt%) were all 99% or more. The fatty acid constitutions of the phosphatidylcholine products in the fatty acid compositions obtained in Examples 1 to 9 were tested, and the results were shown in Table 1 .

**Table 1 Fatty acid content in the phosphatidylcholine products**

| Kinds of fatty acids (% area) | 1 | 2 | 3 | 4 | 5 | 6 | 7 | 8 | 9 |
|---|---|---|---|---|---|---|---|---|---|
| C14:0 | - | - | 0.04 | - | - | - | - | 0.32 | - |
| C16:0 | 0.1 | 0.08 | 0.04 | 0.06 | 0.05 | 0.11 | 0.15 | 1.93 | 0.19 |
| C16:4 n-1 | - | - | - | - | - | 0.04 | 0.06 | - | - |
| C16:1 n-7 | 0.04 | 0.08 | 0.07 | 0.05 | 0.07 | 0.05 | 0.08 | 0.83 | 0.05 |
| phytanic acid | - | - | 0.11 | - | - | - | - | 0.11 | 0.04 |
| C18:0 | 0.04 | - | - | 0.05 | 0.03 | - | 0.05 | 0.27 | - |
| C18:1 n-9 | 0.23 | 0.41 | 0.05 | 0.07 | 0.03 | 0.19 | 0.27 | 3.21 | 0.18 |
| C18:1 n-7 | 0.09 | 0.17 | - | - | - | 0.05 | 0.08 | 0.99 | 0.06 |
| C18:2 n-9 | 0.03 | 0.06 | - | - | - | - | - | 0.08 | - |
| C18:2 n-6 | 0.32 | 0.67 | 0.21 | 0.07 | 0.12 | 0.06 | 0.08 | 0.31 | 0.08 |
| C18:2 n-4 | 0.38 | 0.28 | 0.04 | - | - | - | - | 0.14 | 0.24 |
| C18:3 n-6 | 0.17 | 0.1 | 0.29 | - | - | - | - | 0.06 | - |
| C18:3 n-4 | 0.12 | 0.07 | 0.13 | - | - | - | - | 0.04 | - |
| C18:3 n-3 | 0.29 | 0.46 | 0.28 | 0.08 | 0.05 | 0.07 | 0.07 | 0.20 | 0.08 |
| C18:4 n-3 | 2.07 | 0.9 | 3.09 | 0.69 | 0.14 | 0.54 | 0.66 | 0.43 | 0.83 |
| C18:4 n-1 | 0.19 | 0.11 | 0.25 | 0.06 | - | 0.03 | - | - | 0.07 |
| C20:1 n-9 | 0.07 | - | - | 0.06 | - | - | 0.03 | 0.7 | - |
| C20:2 n-9 | 0.06 | - | - | - | - | - | - | - | - |
| C20:3 n-6 | 0.2 | 0.53 | 0.09 | - | - | - | - | - | - |
| C20:4 n-6 | 3.67 | 4.38 | 2.05 | 0.78 | 1.50 | 0.81 | 1.86 | 0.87 | 0.93 |
| C20:4 n-3 | 1.44 | 2.63 | 1.09 | 0.97 | 0.57 | 0.99 | 1.34 | 1.17 | 0.98 |
| C20:5 n-3 | 73.01 | 55.47 | 44.14 | 25.39 | 8.91 | 23.74 | 21.11 | 18.05 | 27.65 |
| C21:5 n-3 | 1.07 | 2.33 | 1.99 | 2.34 | 0.57 | 2.40 | 2.83 | 2.03 | 2.26 |
| C22:4 | 0.07 | 0.23 | 0.05 | 0.05 | 0.2 | 0.08 | 0.2 | 0.14 | 0.06 |
| C22:5 n-6 | 0.51 | 0.66 | 1.16 | 1.81 | 1.45 | 2.03 | 3.01 | 1.31 | 1.84 |
| C22:5 n-3 | 1.42 | 4.24 | 6.36 | 10.71 | 8.55 | 11.36 | 6.66 | 10.33 | 10.3 |
| C22:6 n-3 | 11.79 | 23.5 | 36.33 | 54.58 | 74.13 | 54.32 | 58.27 | 51.02 | 51.54 |
| Maxim um undetermined fatty acid | 0.24 | 0.28 | 0.28 | 0.58 | 0.9 | 0.68 | 0.53 | 0.44 | 0.66 |
| Total undetermined fatty acids | 1.2 | 1.56 | 1.47 | 1.45 | 2.26 | 2.22 | 2.23 | 4.68 | 1.4 |
| Other impurities | 1.42 | 1.08 | 0.67 | 0.73 | 1.37 | 0.9 | 0.97 | 0.77 | 1.21 |
| EPA+DHA | 84.8 | 78.97 | 80.47 | 79.77 | 83.04 | 78.06 | 79.38 | 69.07 | 79.19 |
| EPA:DHA | 6.19 | 2.36 | 1.21 | 0.47 | 0.12 | 0.44 | 0.36 | 0.35 | 0.54 |
| n-3+n-6 | 95.96 | 95.87 | 97.08 | 97.42 | 95.99 | 96.33 | 95.89 | 85.78 | 96.48 |
| Other polyunsaturated | 0.85 | 0.75 | 0.47 | 0.11 | 0.2 | 0.15 | 0.26 | 0.4 | 0.38 |
| fatty acids | | | | | | | | | |
| saturated fatty acid | 0.14 | 0.08 | 0.19 | 0.11 | 0.08 | 0.11 | 0.2 | 2.64 | 0.23 |
| monounsaturated fatty acid | 0.43 | 0.66 | 0.12 | 0.18 | 0.1 | 0.29 | 0.46 | 5.73 | 0.29 |

| | | | | | | | | | |
|---|---|---|---|---|---|---|---|---|---|
| Note: "-" means non-detected. | | | | | | | | | |

### Example 10 Pharmacological experiment of the fatty acid compositions in Examples 1-9

### I. Materials and methods

1. Animals: C57BL/6J mice, male (18-20g); 10 mice per group; purchased from Beijing Huafukang Biotechnology Co., Ltd.; license number: SCXK (Beijing) 2014-0004.
2. Grouping:
   (1) Normal control group (control group): 0.5wt% sodium carboxymethyl cellulose was administered intragastrically.
   (2) IBD model group (model group): 0.5wt% sodium carboxymethyl cellulose was administered intragastrically.
   (3) Positive drug sulfasalazine group (SASP group): Shanghai Sinepharm Factory Co., Ltd. (batch number: 036151102); dosage: 500mg/kg, prepared with 0.5wt% sodium carboxymethyl cellulose, stored at 4°C, administered intragastrically once a day.
   (4) Test group: the fatty acid compositions of Examples 1 to 9, dosage: 500 mg/kg, prepared with 0.5wt% sodium carboxymethyl cellulose, stored at 4°C, administered intragastrically once a day.
3. Experimental method

For the convenience of experimental operation, two experiments were conducted. The fatty acid compositions of Examples 1 to 5 were used for the first experiment; the fatty acid compositions of Examples 6 to 9 were used for the second experiment. Both experiments were operated according to the following methods.

C57BL/6J mice were adaptively fed in an SPF animal room (laboratory animal license number: SYXK (Beijing) 2014-0023) for one week, and then randomly grouped. The mice in the model group and the administration groups were constructed as an IBD mouse model with DSS (MP, CA9011-18-1, US) by entrusting to the Institute of Materia Medica, Chinese Academy of Medical Sciences. The mice in the normal control group were not treated. The normal control group (control group) and model group (model group) were administered with 0.5wt% sodium carboxymethyl cellulose intragastrically, once a day, with an administration volume of 10 mL/kg. The SASP group and the test group were administered intragastrically according to the above experimental scheme, once a day. The animals in the model group showed obvious listlessness, reduced activity, body weight loss, sparse stools, hematochezia and other typical IBD lesions after 7 days of modeling, and the experiment can be terminated. The animals in each group were sacrificed, and each evaluation indicator of colitis was tested (see the section of Experiment results), and the anti-IBD pharmacodynamic activity of each test sample was evaluated comprehensively.

### II. Experimental results

The results were shown in Table 2. The experimental results showed that, compared with the normal control group, the colonic mucosa of mice in the model group modeled by DSS was significantly shed. The inflammation implicated the mucosa and the submucosal lamina propria, and crypts were destroyed; the infiltration of a large number of inflammatory cells, which are mainly lymphocytes and neutrophils, can be seen at the inflammatory lesion site; inflammatory ulcer formation, bleeding and blood cell exudation can be seen in local areas with significant inflammation; inflammatory fibrous tissue hyperplasia can be seen at the bottom of the ulcer foci; a large number of red blood cells and exudates can be seen in the intestinal cavity. The positive drug SASP can effectively improve the colon tissue damage of DSS-induced IBD mouse model animals to a certain extent, showing that the structure of the intestinal mucosa was basically maintained normal, and the epithelial cells were occasionally arranged disorderly and loss of polarity; scattered superficial ulcers were formed; mild edema and inflammatory cell infiltration occurred in the submucosa and muscle layer; no obvious serosal layer destruction and mucosal hemorrhage were observed. Compared with the model group, the test groups of Example 1, Example 2, Example 3 and Example 5 showed no significant decrease in histopathological score, and there was no statistically significant difference. The test group of Example 4 has relatively obvious anti-IBD activity, which is manifested as a decrease in histopathological score and an increase in DAI comprehensive index inhibition rate. Compared with the model group, the statistical difference is very significant (p<0.01). The histopathological score was assessed from intestinal epithelial mucosal injury and ulcer formation, edema, lymphocyte/monocyte/plasma cell infiltration, neutrophil infiltration, eosinophil infiltration and other indicators. The lower the histopathological score was, the closer the colon tissue was to the normal physiological state of the animal. Table 2 showed the effect of the test groups of Examples 1 to 5 on the inhibition rate of the disease comprehensive index DAI and histopathological scores of IBD mice verified in Example 10 of the application.

**Table 2 DAI comprehensive index inhibition rate and histopathological score**

| Group | DAI comprehensive index inhibition rate (%) | histopathological score |
|---|---|---|
| normal control group | - | 0.30±0.67 |
| IBD model group | 0 | 8.80±1.44** |
| Positive drug group | 23.08^{#} | 7.25±2.14^{#} |
| Test group of Example 1 | 8.33 | 8.13±1.27 |
| Test group of Example 2 | 15.39 | 7.89±1.45 |
| Test group of Example 3 | -2.69 | 7.70±2.11 |
| Test group of Example 4 | 48.08^{##} | 5.40±1.43^{##} |
| Test group of Example 5 | 0 | 8.38±0.99 |

| | | |
|---|---|---|
| Note: **p<0.01 vs. Con; ^{#}p<0.05, ^{##}p<0.01 vs. Mod. | | |

The experimental results showed that, compared with the normal control group (con), disease comprehensive index DAI of the model group (mod) animal increased significantly, and the statistical difference was very significant, indicating that the model was successfully built. Compared with the model group, the positive drug group (SASP group) and the test group of Example 4 can reduce the disease comprehensive index DAI score of the experimental animal significantly, and the difference was statistically very significant. There was no significant improvement effect in the test groups of Examples 1 to 3 and the test group of Example 5, and there was no difference in statistical test. The DAI score was evaluated from the degree of body weight loss, stool character, hematochezia and other indicators of animal. The lower the DAI score was, the closer the animal was to the normal physiological state.

The DAI scoring criteria were as follows:

| Score | Percentage of body weight loss (%) | Stool character | Degree of hematochezia |
|---|---|---|---|
| 0 | 0 | Normal | Normal |
| 1 | 1~ | Loose(+) | Occult blood positive(+) |
| 2 | 5~ | Loose(++) | Occult blood positive(++) |
| 3 | 10~ | Sparse stool(+) | Bloody stool with naked eyes(+) |
| 4 | > 15 | Sparse stool(++) | Bloody stool with naked eyes(++) |
| ~ Referring to the right open interval, for example, 1~ refers to [1%, 5%) | | | |

| | | | |
|---|---|---|---|
| Note: ① Normal stool: formed stool; ② Loose stool: mushy, semi-formed stool that does not adhere to the anus; ③ Sparse stool: watery stool. | | | |

The histopathology scoring criteria table was as follows:

| Scoring criteria | 0 | 1 | 2 | 3 |
|---|---|---|---|---|
| Epithelial injury and ulceration | None | Submucosa/ erosion | Muscle layer/ulcers | Serosal layer/heavy |
| Edema | None | Mild | Moderate | Heavy |
| Lymphocyte, monocyte, plasma cell infiltration | None | Submucosa/mild | Muscle layer/moderate | Serosal layer/heavy |
| Neutrophil infiltration | None | Submucosa/mild | Muscle layer/moderate | Serosal layer/heavy |
| Eosinophil infiltration | None | Submucosa/mild | Muscle layer/moderate | Serosal layer/heavy |

The experimental results of the test groups of Examples 6-9 showed that, compared with the normal group, the colonic mucosa of mice in the model group modeled by DSS was significantly shed. The inflammation implicated the mucosa and the submucosal lamina propria, and crypts were destroyed; the infiltration of a large number of inflammatory cells, which are mainly lymphocytes and neutrophils, can be seen at the inflammatory lesion site; inflammatory ulcer formation, bleeding and blood cell exudation can be seen in local areas with significant inflammation; inflammatory fibrous tissue hyperplasia can be seen at the bottom of the ulcer foci; a large number of red blood cells and exudates can be seen in the intestinal cavity (DAI comprehensive index inhibition rate was 0%, histopathological score was 8.45±0.62, statistically significant difference, p<0.01 vs. Con). The positive drug SASP can slightly improve the colon tissue damage of DSS-induced IBD mouse model animals, which was manifested by the improvement of intestinal mucosal structure destruction, the alleviation of epithelial cell disorder arrangement and loss of polarity; scattered superficial ulcer formation; moderate edema and inflammatory cell infiltration in the submucosa and muscle layer; alleviation of the serosal layer destruction and mucosal hemorrhage. Test groups of Example 6, Example 7, Example 8, and Example 9 all had obvious anti-IBD activity (DAI comprehensive index inhibition rates were all higher than 25%, histopathological scores were all lower than 6, statistically significant difference, p<0.01 vs. Mod), and can improve the damage of the colon tissue in the model animal to a certain extent, which was manifested by the alleviation of edema, inflammation, exudation and infiltration and the like of the mucosal layer, submucosa, and muscle layer to a certain extent, and the activities thereof were greater than the positive drug.

### Examples 11 to 13

For further investigating the effect of the total content of EPA and DHA in the fatty acid composition on the anti-IBD activity of the composition, the inventors used the methods of Examples 1 to 9 to prepare the fatty acid composition comprising the phosphatidylcholine products with the fatty acid constitutions in following Table 3 from the commercially available ethyl ester-type fish oils, and the contents of phosphatidylcholine products (wt%) in these compositions obtained in Examples 11-13 were all 99% or more.

**Table 3 Fatty acid content in the phosphatidylcholine products**

| Kinds of fatty acids (% area) | 11 | 12 | 13 |
|---|---|---|---|
| C14:0 | 0.253 | 0.066 | 0.017 |
| C16:0 | 17.649 | 0.175 | 0.075 |
| C16:4 n-1 | - | 0.052 | 0.025 |
| C16:1 n-7 | 0.153 | 0.571 | 0.027 |
| phytanic acid | 0.029 | - | - |
| C18:0 | 0.316 | 0.037 | 0.049 |
| C18:1 n-9 | 7.438 | 2.601 | 0.055 |
| C18:1 n-7 | 0.156 | 0.789 | - |
| C18:2 n-9 | - | 0.083 | - |
| C18:2 n-6 | 1.443 | 0.277 | 0.321 |
| C18:2 n-4 | 0.290 | 0.120 | - |
| C18:3 n-6 | - | 0.052 | 0.038 |
| C18:3 n-4 | - | 0.011 | - |
| C18:3 n-3 | 0.051 | 0.191 | 0.031 |
| C18:4 n-3 | 0.109 | 0.594 | 0.352 |
| C18:4 n-1 | - | 0.081 | 0.050 |
| C20:1 n-9 | 0.198 | 0.611 | 0.024 |
| C20:2 n-9 | - | 0.216 | - |
| C20:3 n-6 | 0.133 | 0.08 | 0.034 |
| C20:4 n-6 | 1.179 | 0.829 | 1.113 |
| C20:4 n-3 | 0.840 | 1.181 | 0.540 |
| C20:5 n-3 | 15.584 | 20.278 | 25.658 |
| C21:5 n-3 | 1.588 | 2.118 | 1.597 |
| C22:4 | 0.302 | 0.137 | 0.095 |
| C22:5 n-6 | 1.488 | 1.179 | 0.635 |
| C22:5 n-3 | 7.078 | 9.646 | 2.623 |
| C22:6 n-3 | 40.132 | 51.477 | 64.528 |
| Total undetermined fatty acids | 2.525 | 5.602 | 1.746 |
| Other impurities | 1.066 | 0.946 | 0.367 |
| EPA+DHA | 55.716 | 71.755 | 90.186 |
| EPA:DHA | 0.388 | 0.394 | 0.398 |
| saturated fatty acid | 18.247 | 0.278 | 0.141 |
| monounsaturated fatty acid | 7.945 | 4.572 | 0.106 |
| n-3 | 65.38 | 85.49 | 95.33 |
| n-6 | 4.243 | 2.417 | 2.141 |

| | | | |
|---|---|---|---|
| Note: "-" means non-detected. | | | |

### Example 14 Pharmacological experiment of the fatty acid compositions in Examples 11-13

### I. Materials and methods

1. Animals: C57BL/6J mice, male (18-20g); 10 mice per group; purchased from Beijing Huafukang Biotechnology Co., Ltd.; license number: SCXK (Beijing) 2014-0004.
2. Grouping:
   (1) Normal control group (control group): 0.5wt% sodium carboxymethyl cellulose was administered intragastrically.
   (2) IBD model group (model group): 0.5wt% sodium carboxymethyl cellulose was administered intragastrically.
   (3) Positive drug sulfasalazine group (SASP group): sulfasalazine enteric-coated tablets; dosage: 500 mg/kg, prepared with 0.5 wt% sodium carboxymethyl cellulose; stored at 4°C and administered intragastrically once a day.
   (4) Test group: the fatty acid compositions of Examples 11 to 13, dosage: 500 mg/kg, prepared with 0.5 wt% sodium carboxymethyl cellulose; stored at 4°C and administered intragastrically once a day.
3. Experimental method

C57BL/6J mice were adaptively fed in an SPF animal room (laboratory animal license number: SYXK (Beijing) 2014-0023) for one week, and then randomly grouped. The mice in the model group and the administration groups were constructed as an IBD mouse model with DSS (MP, CA9011-18-1, US, keeping away from light) by entrusting to the Institute of Materia Medica, Chinese Academy of Medical Sciences. The mice in the normal control group were not treated. The normal control group and model group were administered with 0.5wt% sodium carboxymethyl cellulose intragastrically, once a day. The SASP group and the test group were administered intragastrically according to the above experimental scheme, once a day. The animals in the model group showed obvious listlessness, reduced activity, body weight loss, sparse stools, hematochezia and other typical IBD lesions after 6 days of modeling, and the experiment was terminated. The animals in each group were sacrificed, and each evaluation indicator of colitis (see the section of Experiment results) was tested, and the anti-IBD pharmacodynamic activity of each test sample was evaluated comprehensively.

### II. Experimental results

Table 4 showed the effect of the test groups of Examples 11 to 13 on the inhibition rate of the disease comprehensive index DAI of IBD mice verified in Example 14 of the application.

**Table 4 DAI disease comprehensive index inhibition rate**

| Group | DAI comprehensive index inhibition rate (%) |
|---|---|
| Normal control group | - |
| IBD model group | 0 |
| Positive drug group | 38.7^{#} |
| Test group of Example 11 | 32.06^{##} |
| Test group of Example 12 | 57.3^{##} |
| Test group of Example 13 | 30.7^{##} |

| | |
|---|---|
| Note: ^{#}p<0.05, ^{##}p<0.01 vs. Mod. | |

The experimental results showed that, compared with the model group, all of the test groups of Examples 11, 12 and 13 can significantly increase the inhibition rate of the disease activity index of experimental animals. The test groups of Examples 11, 12 and 13 were compared, statistical analyses were performed on the test group of Example 12 with the test groups of Examples 11 and 13, respectively, and the results showed that the statistical difference was very significant. The disease activity index inhibition rate of the test group of Example 12 was significantly higher than that of the test groups of Examples 11 and 13, indicating that the efficacy of the test group of Example 12 was significantly superior to the test group of Example 11 and the test group of Example 13.

### Example 15 Effect on TNBS-induced rat IBD model

1. Animals: SD rats, SPF grade, male (122.3 to 152.6 g), 15 rats per group.
2. Grouping:
   (1) Normal control group (control group): 0.5wt% sodium carboxymethyl cellulose (CMC-Na) was administered intragastrically.
   (2) IBD model group (model group): 0.5wt% sodium carboxymethyl cellulose was administered intragastrically.
   (3) Positive drug sulfasalazine group (SASP group): sulfasalazine enteric-coated tablets, dosage: 600mg/kg, prepared with 0.5% sodium carboxymethyl cellulose; administered intragastrically once a day.
   (4) Test group: The fatty acid composition of Example 4, which was divided into three groups of 1, 2 and 3 according to the dosage of 150, 300 and 600 mg/kg, and prepared with 0.5wt% sodium carboxymethyl cellulose; administered intragastrically once a day.
3. Experimental method: After being fasted but water allowed for about 24 hours, rats in each group were anesthetized by intraperitoneal injection of 3% sodium pentobarbital solution, and a plastic tube with a diameter of 2 mm and a length of about 12 cm was gently inserted into the rat body via the anus for about 8cm deep. The ethanol solution containing TNBS was slowly injected with the dose of TNBS 75 mg/kg and the administration volume of 3 mL/kg body weight, and then about 0.1 mL of air was injected to discharge the medicine remained in the plastic tube into intestine. After the administration, the plastic tube was slowly pulled out, the anus was pinched with hand, and the tail of the rat was lifted and kept upside down for about 30s, so that the modeling agent can fully penetrate into the intestinal cavity of the rat. The normal control group was injected with the same amount of normal saline. Based on the body weight thereof, 75 TNBS modeled rats were randomly divided into model group, positive drug group (sulfasalazine 600 mg/kg body weight), test groups 1 to 3, 15 rats/group, and a normal control group was set up, in which 15 rats were included. On the second day after modeling, the corresponding test samples were administered intragastrically. The normal control group and the model group were administered with 0.5wt% CMC-Na once a day for 5 consecutive days. DAI activity score was performed on days 1 and 3 of administration and 24 hours after the last administration. Intestinal mucosal damage was observed 24 hours after the last administration for CMDI scoring. The colon tissue was taken for histopathological examination.
4. Experimental results: ① Dark red filth was observed near the anus of the rats in the model group after TNBS modeling, and some animals excreted obvious sparse stools and bloody stools. The body weight of d6 and the weight gain during the administration period were lower (p<0.05), DAI score values of each time point of d1, d3 and d6, and CMDI score value of d6 were higher (p<0.05), and the length of colon + rectum was shorter (p<0.05). Histopathological examination showed that intestinal wall inflammatory cell infiltration, intestinal crypt structure destruction and mucosal fibrosis were found in different degrees in 6 cases of 15 cases, mucosal ulcers and edema were observed in some animals. ② Positive drug group: the symptoms of sparse stools and bloody stools of each group were alleviated until disappeared during the administration period. The weight gain during the administration period was lower (p<0.05), and DAI and CMDI score values of d6 were lower (p<0.05). Histopathological examination showed histological changes of the colon in 5 cases of15 cases, and the lesion degree was significantly reduced compared with the model group. ③ Test groups 1 to 3: the symptoms of sparse stools and bloody stools of animals in each group alleviated day by day until disappeared during the administration period. DAI score value of rats in test group 3 at time point of d6 was lower (p<0.05), and CMDI score values of rats in test groups 1 to 3 all had a decreasing trend. Histopathological examination found that the degree of colon histological lesions in the rats of test groups 1 to 3 was alleviated compared to the model group.

### Example 16 Effect on OXZ-induced mouse IBD model

1. Animals: Balb/c mice, male (20-22g); 10 mice per group.
2. Grouping:
   (1) Normal control group (control group): 0.5wt% sodium carboxymethyl cellulose was administered intragastrically.
   (2) IBD model group (model group): 0.5wt% sodium carboxymethyl cellulose was administered intragastrically.
   (3) Positive drug sulfasalazine group (SASP group): Dosage: 500mg/kg, prepared with 0.5wt% sodium carboxymethyl cellulose; stored at 4°C and administered intragastrically once a day.
   (4) Test group: The fatty acid compositions of Example 4, which was divided into two groups (test group 1 and test group 2) according to the dosages of 500 mg/kg and 1000 mg/kg, prepared with 0.5 wt% sodium carboxymethyl cellulose; stored at 4°C and administered intragastrically once a day.
3. Experimental method
   Balb/c mice were adaptively fed in an SPF animal room for one week and then grouped randomly. The mice in the model group and the administration groups were constructed as an IBD mouse model with OXZ by entrusting to the Institute of Materia Medica, Chinese Academy of Medical Sciences. Then, the administration was performed for 6 consecutive days; the normal control group was not treated. The normal control group and the model group were intragastrically administered with 0.5wt% sodium carboxymethylcellulose, once a day. SASP group and test groups 1 to 2 were administrated intragastrically according to the above experimental scheme, once a day. Animals in each group were sacrificed on day 6 of modeling, and various indicators related to colitis were tested (see the section of Experimental results).
4. Experimental results
   Table 5 showed the effect of the fatty acid composition in Example 4 of the application on the inhibition rate of the disease comprehensive index DAI and the histopathological score of IBD mice.

**Table 5 DAI comprehensive index inhibition rate and histopathological score**

| Group | DAI composite index inhibition rate (%) | histopathological score |
|---|---|---|
| Normal control group | - | 0.60±0.16 |
| IBD model group | - | 9.38±0.56** |
| Positive drug group | 50 | 4.38±0.42^{##} |
| Test group 1 (500mg/kg) | 58.85^{#} | 6.00±0.46^{##} |
| Test group 2 (1000mg/kg) | 67.71⁴ | 4.43±0.62^{##} |

| | | |
|---|---|---|
| Note : **p<0.01 vs. Con; ^{#}p<0.05, ^{##}p<0.01 vs. Mod. | | |

The experimental results showed that compared with the normal control group (con), the animal disease comprehensive index DAI of the model group (mod) increased significantly, and the statistical difference was very significant, indicating that the model was successfully built. Compared with the model group, test groups 1 to 2 can significantly reduce the score of the disease comprehensive index DAI of experimental animals, and the difference was statistically significant.

### Example 17 Pharmacokinetic experiment

Administration route: single intragastric administration

Test drug: medium-chain triglycerides (MCT) were added to the fatty acid composition of Example 4 to prepare the test products of groups A to C, and the test product of group D was prepared by using the ethyl ester-type fish oil with the same fatty acid constitution as the commercially available Lovaza product.

Test animals: 24 male SD rats qualified for quarantine and adaptation period, with 300-350g of body weight, were taken. PEMS 3.1 software was used to randomly assign them into 4 groups: group A (administration dose 100mg/kg, administration concentration 10 mg/mL, administration volume 10 mL/kg), group B (administration dose 300mg/kg, administration concentration 30 mg/mL, administration volume 10 mL/kg), group C (administration dose 900 mg/kg, administration concentration 90 mg/mL, administration volume 10 mL/kg), group D (administration dose 900 mg/kg, administration concentration 90 mg/ml, administration volume 10 mL/kg).

Administration method: After being fasted but water allowed overnight for at least 12 hours, they were fed high-fat diet the next morning, the food intake within 0.5 hours was recorded, and the test drugs of corresponding concentrations were administered intragastrically according to the above administration scheme after 0.5 hours. They were fasted within 4 hours after the administration, and all fed standard diet after 4 hours, and the food intake within 10 hours after the administration needed to be recorded (the food intake was measured every 2 hours).

Test process: The time points of blood sampling were set as follows: Baseline (4 points): -24h, -16h, -8h, 0h before administration; after administration (11 points): venous blood of 1h, 2h, 4h, 6h, 7h, 8h, 10h, 12h, 24h, 48h, 72h after administration; 15 points were sampled in total. 0.2 ml of blood was collected from the retroorbital venous plexus, anticoagulated with EDTA-2K, and centrifuged at 4°C under 1700xg for 10 min, and the plasma was separated, and stored at - 80°C for testing.

Sample analysis: The plasma concentration of EPA total acid, DHA total acid, EPA free acid (EPA-FA), and DHA free acid (DHA-FA) in plasma were determined by LC/MS/MS method.

Conclusion: The results of area under the plasma concentration-time curve (AUC) showed that the fatty acid composition comprising the phosphatidylcholine product of the application had better absorption, compared with the control drug ethyl ester-type fish oil at the same dosage. The statistical analysis results of each pharmacokinetic parameter showed that the dosage of the fatty acid composition comprising the phosphatidylcholine product of the application was correlated with the drug system exposure.

Described above are only the preferred embodiments of the application. It should be pointed out that for those skilled in the art, without departing from the principles of this application, several improvements and modifications may be made, and these improvements and modifications also should be regarded as falling within the protection scope of this application.

## Claims

1. . A phosphatidylcholine product represented by Formula (I): 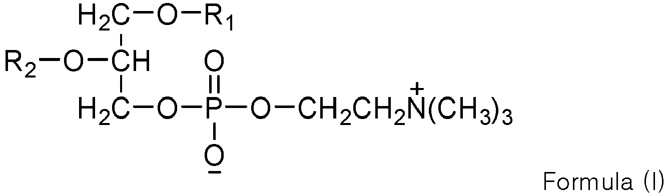 in Formula (I), R₁ and R₂ are each independently selected from residues of saturated or unsaturated fatty acids having 12 or more carbon atoms; and EPA residue(s) and DHA residue(s) are comprised in R₁ and R₂, an area ratio between EPA and DHA is (about 0.2 to about 1):1, preferably (about 0.25 to about 0.65):1, more preferably (about 0.25 to about 0.6):1 .

2. . The phosphatidylcholine product according to claim 1, wherein the area ratio between EPA and DHA is (about 0.33 to about 0.6):1.

3. . The phosphatidylcholine product according to claim 1 or 2, wherein R₁ and R₂ are each independently selected from residues of saturated or unsaturated fatty acids having 12 to 24, preferably 14 to 22 carbon atoms.

4. . The phosphatidylcholine product according to any one of claims 1 to 3, wherein an area percentage of EPA to a total amount of fatty acids in the phosphatidylcholine product represented by Formula (I) is about 10% to about 40%, preferably about 15% to about 35%, more preferably about 15% to about 32%, further preferably about 18% to about 32%.

5. . The phosphatidylcholine product according to any one of claims 1 to 4, wherein an area percentage of DHA to a total amount of fatty acids in the phosphatidylcholine product represented by Formula (I) is about 40% to about 70%, preferably about 40% to about 65%, more preferably about 45% to about 65%.

6. . The phosphatidylcholine product according to any one of claims 1 to 5, wherein the area percentage of EPA to the total amount of fatty acids in the phosphatidylcholine product represented by Formula (I) is about 10% to about 35%, and the area percentage of DHA to the total amount of fatty acids in the phosphatidylcholine product represented by Formula (I) is about 40% to about 70%; preferably, the area percentage of EPA to the total amount of fatty acids in the phosphatidylcholine product represented by Formula (I) is about 15% to about 32%, and the area percentage of DHA to the total amount of fatty acids in the phosphatidylcholine product represented by Formula (I) is about 40% to about 65%; more preferably, the area percentage of EPA to the total amount of fatty acids in the phosphatidylcholine product represented by Formula (I) is about 18% to about 32%, and the area percentage of DHA to the total amount of fatty acids in the phosphatidylcholine product represented by Formula (I) is about 45% to about 65%; further preferably, the area percentage of EPA to the total amount of fatty acids in the phosphatidylcholine product represented by Formula (I) is about 18% to about 32%, and the area percentage of DHA to the total amount of fatty acids in the phosphatidylcholine product represented by Formula (I) is about 50% to about 60%.

7. . The phosphatidylcholine product according to any one of claims 1 to 6, wherein an area percentage of saturated fatty acids to a total amount of fatty acids in the phosphatidylcholine product represented by Formula (I) is not more than 20%, preferably not more than 5%, more preferably about 0.01% to about 5%, and further preferably about 0.05% to about 3%, and an area percentage of monounsaturated fatty acids to a total amount of fatty acids in the phosphatidylcholine product represented by Formula (I) is not more than 10%, preferably not more than 8%, more preferably about 0.01% to about 6%.

8. . The phosphatidylcholine product according to any one of claims 1 to 7, wherein an area percentage of n-3 fatty acids to a total amount of fatty acids in the phosphatidylcholine product represented by Formula (I) is about 60% or more, preferably about 65% to about 99%, more preferably about 65% to about 96%, and relative to the n-3 fatty acids, an area percentage of EPA is about 15% to about 40%, preferably about 20% to about 30%, more preferably about 21% to about 30%.

9. . The phosphatidylcholine product according to any one of claims 1 to 8, wherein an area percentage of n-3 fatty acids to a total amount of fatty acids in the phosphatidylcholine product represented by Formula (I) is about 60% or more, preferably about 65% to about 99%, more preferably about 65% to about 96%, and relative to the n-3 fatty acids, an area percentage of DHA is about 45% to about 75%, preferably about 50% to about 75%, more preferably about 55% to about 70%.

10. . The phosphatidylcholine product according to any one of claims 1 to 9, wherein an area percentage of DPA to a total amount of fatty acids in the phosphatidylcholine product represented by Formula (I) is not more than 15%, preferably about 2% to about 15%, more preferably about 5% to about 12%.

11. . The phosphatidylcholine product according to any one of claims 1 to 10, wherein an area percentage of n-3 fatty acids to a total amount of fatty acids in the phosphatidylcholine product represented by Formula (I) is about 60% or more, preferably about 65% to about 99%, more preferably about 65% to about 96%, and relative to the n-3 fatty acids, an area percentage of DPA is about 2% to about 20%, preferably about 2% to about 15%, more preferably about 7% to about 15%, further preferably about 7% to about 13%.

12. . A fatty acid composition comprising a phosphatidylcholine product represented by Formula (I) according to any one of claims 1 to 11 : 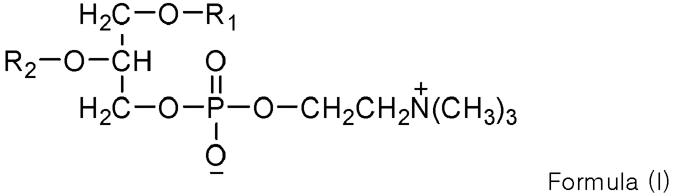 in Formula (I), R₁ and R₂ are each independently selected from residues of saturated or unsaturated fatty acids having 12 or more carbon atoms; and EPA residue(s) and DHA residue(s) are comprised in R₁ and R₂, wherein an area ratio between EPA and DHA is (about 0.2 to about 1): 1, preferably (about 0.25 to about 0.65):1, more preferably (about 0.25 to about 0.6):1.

13. . The composition of claim 12, wherein the area ratio between EPA and DHA is (about 0.33 to about 0.6):1.

14. . The composition according to claim 12 or 13, wherein an area percentage of EPA to a total amount of fatty acids in the phosphatidylcholine product represented by Formula (I) is about 10% to about 35%, and an area percentage of DHA to a total amount of fatty acids in the phosphatidylcholine product represented by Formula (I) is about 40% to about 70%; preferably, the area percentage of EPA to the total amount of fatty acids in the phosphatidylcholine product represented by Formula (I) is about 15% to about 32%, and the area percentage of DHA to the total amount of fatty acids in the phosphatidylcholine product represented by Formula (I) is about 40% to about 65%; more preferably, the area percentage of EPA to the total amount of fatty acids in the phosphatidylcholine product represented by Formula (I) is about 18% to about 32%, and the area percentage of DHA to the total amount of fatty acids in the phosphatidylcholine product represented by Formula (I) is about 45% to about 65%; further preferably, the area percentage of EPA to the total amount of fatty acids in the phosphatidylcholine product represented by Formula (I) is about 18% to about 32%; and the area percentage of DHA to the total amount of fatty acids in the phosphatidylcholine product represented by Formula (I) is about 50% to about 60%.

15. . A phosphatidylcholine product represented by Formula (I) according to any one of claims 1 to 11 or a fatty acid composition according to any one of claims 12 to 14 for use in preventing or treating inflammatory bowel disease; preferably, the inflammatory bowel disease is selected from ulcerative colitis or Crohn's disease.

16. . A method for preparing a fatty acid composition of any one of claims 12 to 14, comprising subjecting free fatty acids to a condensation reaction with glycerol phosphatidylcholine, and purifying resulted reaction product to obtain the fatty acid composition;
preferably, the method further comprises hydrolyzing an ester-type fish oil to obtain the free fatty acids;
more preferably, the condensation reaction is performed in the presence of alkali, a reaction solvent and a condensation agent, and the reaction solvent is selected from dichloromethane, chloroform, diethylformamide, tetrahydrofuran, N,N-dimethyl formamide or dioxane, the alkali is selected from sodium carbonate, potassium carbonate, potassium acetate, triethylamine, 4-dimethylaminopyridine or N,N-diisopropylethylamine, and the condensation agent is selected from 2-(7-azabenzotriazole)-N, N, N', N'-tetramethyluronium hexafluorophosphate, benzotriazole-N,N,N',N'-tetramethyluronium hexafluorophosphate or carbodiimide.
